# FASCICULE DE BREVET EUROPEEN

(11) **EP 1 176 132 B1**
(45) Date de publication et mention de la délivrance du brevet: **28.02.2007**
(21) Numéro de dépôt: 01402018.4
(22) Date de dépôt: 26.07.2001
(51) Int. Cl.: C07C 41/06, C07C 43/04, C07C 41/36, C07C 41/44

(54) **Procédé et dispositif d'éthérification d'oléfines d'une charge d'hydrocarbures et leur utilisation**
Verfahren und Vorrichtung zur Etherifizierung von Olefinen enthaltenden Kohlenwasserstoffeinsätzen sowie ihre Verwendung
Process and apparatus for the etherification of olefins in a hydrocarbon charge and their use

(30) Priorité: 26.07.2000 FR 0009769
(43) Date de publication de la demande: 30.01.2002
(73) Titulaire: TotalFinaElf France, 92800 Puteaux (FR)
(72) Inventeur: Vermeiren, Walter Josephus, 3530 Houthalen-Helchteren (BE); Martins Mendes Cerejo, Francisco Anton, 7190 Ecaussinnes d'Enghien (BE)
(74) Mandataire: Jolly, Jean-Pierre

(56) Documents cités:
- WO-A-97/45392
- GB-A- 2 265 145
- US-A- 5 120 881
- US-A- 5 352 848
- US-A- 5 569 790

## Description

La présente invention concerne un procédé d'éthérification d'oléfines contenues dans une charge d'hydrocarbures, par mise en contact de cette charge avec au moins un alcool, dans des conditions d'éthérification, en présence d'un catalyseur de type acide. L'invention a plus particulièrement pour objet un tel procédé appliqué à une charge contenant des composés azotés susceptibles de désactiver les sites acides du catalyseur.

L'invention concerne également un dispositif pour la mise en oeuvre de ce procédé.

L'invention concerne enfin l'utilisation de ce procédé ou de ce dispositif pour la préparation d'éthers, utilisables par exemple comme additifs pour carburants de véhicules automobiles.

On connaît, en effet, le développement considérable qu'a connu ces dernières années l'utilisation de certains éthers dans les carburants, pour améliorer leur indice d'octane, car ils sont moins nocifs que les dérivés du plomb précédemment employés à cet effet.

Parmi les éthers les plus utilisés dans ce but, on mentionnera, notamment, le méthyl tert-butyl éther (MTBE), l'éthyl tert-butyl éther (ETBE) et le tert-amyl éther (TAME).

Ces éthers peuvent être préparés par réaction d'éthanol ou de méthanol avec l'iso-oléfine appropriée, dans des conditions d'éthérification, en présence d'un catalyseur acide, et l'on utilise comme charge une coupe convenable d'hydrocarbures riche en oléfines, provenant par exemple d'une unité de craquage catalytique à lit fluidisé (FCC) ou encore d'une unité de vapocraquage.

C'est ainsi, par exemple, que le MTBE est préparé à partir d'une coupe d'hydrocarbures riche en isobutène, que l'on fait réagir avec du méthanol, habituellement sur un catalyseur solide tel qu'une résine greffée par des groupements acide sulfonique.

La charge d'hydrocarbures contient en général de très faibles quantités d'impuretés azotées, typiquement des nitriles, mais aussi des amines, imines ou autres hydrocarbures azotés. En raison de leur caractère basique, ces impuretés azotées ont tendance à empoisonner les sites acides du catalyseur d'éthérification, ce qui a pour effet de désactiver progressivement celui-ci.

C'est le cas, par exemple, dans un procédé de synthèse du MTBE, utilisant un catalyseur comprenant un copolymère de styrène et de vinyl benzène sur lequel sont greffés des groupes acide sulfonique, qui sont des sites de type acide de Bronstedt, tandis que dans la charge d'isobutène sont habituellement présents des nitriles, tels que l'acétonitrile, qui sont des bases faibles, qui vont donc réagir avec ces sites acides pour les neutraliser.

Par ailleurs, les conditions réactionnelles de l'éthérification sont favorables à des phénomènes d'hydrolyse totale ou partielle des nitriles pour former en particulier des amides, des amines, de l'ammoniaque. Nettement plus basiques que les nitriles, ces composés réagissent immédiatement avec les sites acides du catalyseur en les neutralisant.

Les réactions aboutissant à la désactivation du catalyseur à la suite d'une hydrolyse de l'acétonitrile sont résumées dans l'équation (1) ci-après :

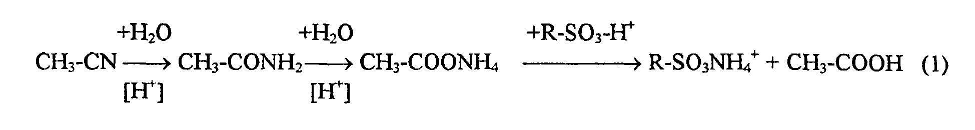

Le catalyseur peut aussi être désactivé à la suite d'une alcoolyse de l'acétonitrile par réaction avec le méthanol, et le mécanisme des réactions est illustré par l'équation (2) ci-après :

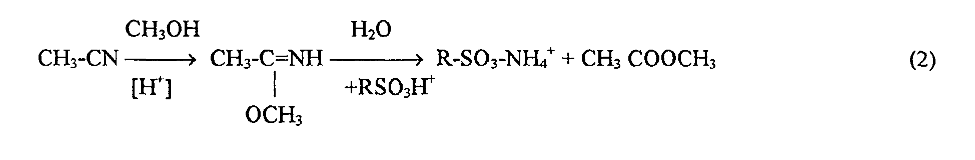

De ces deux réactions, il ressort que les sites acide sulfonique du catalyseur sont convertis en sites ammonium inactifs, du fait de la présence de nitriles dans la charge d'isobutène.

Cet exemple illustre comment, de manière plus générale, les catalyseurs acides utilisés dans les procédés d'éthérification sont désactivés prématurément, du fait de la présence de composés azotés dans les coupes d'oléfines légères à éthérifier.

En effet, les coupes riches en oléfines légères, que l'on utilise typiquement pour la production d'éthers, comprennent souvent une quantité substantielle de composés azotés, pouvant atteindre 100 à 200 p.p.m. pour des coupes provenant d'une unité de craquage catalytique en lit fluidisé (F.C.C.).

Ces composés azotés ne peuvent pas être éliminés par distillation, car ils ont tendance à former des azéotropes avec les hydrocarbures présents. Compte tenu de leur effet néfaste sur les catalyseurs utilisés dans les procédés d'éthérification, on a recherché d'autres moyens pour les séparer de la charge d'hydrocarbures, avant d'introduire celle-ci dans le réacteur d'éthérification.

C'est ainsi que US-A-5 120 881 propose de prétraiter la charge d'éthérification en présence d'une zéolite, de manière à adsorber les composés azotés (nitriles, amines, amides, ammoniaque) qu'elle contient. Pour le même usage, US-A-5 378 250 préconise l'utilisation de milieux particulaires poreux suractivés, tandis que US-A-5 427 689 propose d'utiliser des borates d'aluminium-zirconium. Bien qu'il soit indiqué, dans ce dernier brevet, que l'acétonitrile peut être retiré de la charge hydrocarbonée, aucun taux d'adsorption n'est mentionné.

De même, US-A-5 414 183 décrit un procédé d'élimination des nitriles contenus dans la charge d'hydrocarbures par hydrolyse basique. La charge est mise en contact avec une solution alcaline aqueuse, ce qui convertit les nitriles en ammoniaque, en amides et en acides carboxyliques. La phase hydrocarbure pauvre en composés azotés doit ensuite être lavée à l'eau ou mise en contact avec un adsorbant pour séparer les contaminants restant après l'hydrolyse. Les exemples décrits dans ce brevet montrent que des températures d'hydrolyse relativement élevées, d'environ 100°C, et de longues durées de contact, de l'ordre de 2,9 heures, sont nécessaires pour abaisser de 50 p.p.m. à moins de 0,5 p.p.m. la teneur en nitrile de la charge d'hydrocarbures.

WO 97/45 392 décrit divers procédés pour l'élimination des nitriles, dans un procédé d'éthérification. Dans tous ces procédés, on commence par extraire par un lavage à l'eau, en amont de l'unité d'éthérification, une fraction des nitriles présents dans la charge d'oléfines à éthérifier.

Dans l'un de ces procédés, le mélange résultant d'eau et de nitriles est ensuite réintroduit dans l'effluent de l'unité d'éthérification. Après extraction de l'éther, puis des oléfines et enfin de l'eau, le mélange d'alcool et de nitriles qui subsiste est soumis à une phase d'hydrogénation, en vue d'hydrogéner les nitriles. Les nitriles convertis sont séparés par fractionnement, avant de recycler l'alcool purifié à l'unité d'éthérification.

Ce procédé est toutefois compliqué et coûteux, du fait de la phase d'extraction préalable des nitriles de la charge d'oléfines à éthérifier, qui comprend un lavage à l'eau poussé de l'intégralité de la charge d'oléfines et, par conséquent, exige la mise en oeuvre d'une colonne de lavage de dimensions importantes. Par ailleurs, un tel système d'extraction des nitriles en amont du réacteur d'éthérification, puis de réintroduction de ces nitriles en aval du réacteur, dans les effluents de celui-ci, complique singulièrement le procédé. Enfin, la méthode de purification de recycle d'alcool est relativement coûteuse et peut conduire à une perte d'alcools entraînés hors de l'unité d'éthérification avec les nitriles convertis éliminés par fonctionnement.

Ces procédés de la technique antérieure, destinés à résoudre le problème posé par la présence d'impuretés azotées dans des charges d'oléfines à éthérifier, proposent donc essentiellement un prétraitement de ces charges de manière à en séparer les composés azotés. Toutefois, du fait de la réactivité intrinsèque élevée des oléfines, l'élimination totale des composés azotés est particulièrement difficile et s'accompagne le plus souvent d'une perte en oléfines. Un tel prétraitement s'avère donc souvent fort coûteux, et peu rentable.

Il est usuel, dans les procédés d'éthérification, d'opérer avec un excès d'alcool, afin de favoriser la réaction d'éthérification et d'éviter une éventuelle polymérisation des oléfines contenues dans la charge, l'alcool en excès étant recyclé à la zone réactionnelle. En sortie de celle-ci, on commence généralement par séparer l'éther des effluents réactionnels, puis on procède à un lavage à l'eau pour séparer l'alcool des hydrocarbures restants et à un strippage pour éliminer l'eau de l'alcool.

Les composés azotés étant solubles dans l'alcool, ils sont donc recyclés avec lui à la zone réactionnelle. Ainsi, les composés azotés tournent en circuit fermé dans l'unité d'éthérification, de sorte que la teneur en composés azotés dans la zone réactionnelle augmente progressivement. Bien que la coupe d'hydrocarbures que l'on traite puisse avoir une concentration initiale en composés azotés relativement faible, de l'ordre de quelques centaines de p.p.m., cette concentration peut donc atteindre assez rapidement des niveaux beaucoup plus élevés dans la zone réactionnelle et l'on constate alors une accélération de la désactivation du catalyseur, et une diminution significative des performances et de la rentabilité de l'unité d'éthérification.

Le problème posé par les hautes teneurs en acétonitrile du méthanol recyclé dans le procédé de synthèse du M.T.B.E., a été exposé, par exemple, dans un article de C.R. Marston, dans Fuel Reformulation, Juillet/Août 1994, et il a donc été proposé de ne recycler qu'une partie de ce méthanol, pour éviter de réintroduire l'intégralité des nitriles dans les réacteurs d'éthérification.

C'est ainsi, par exemple, que US-A-5 352 848, qui décrit un procédé d'éthérification d'une charge constituée par un effluent de craquage catalytique en lit fluidisé (F.C.C.), propose d'envoyer à l'unité de F.C.C. une partie au moins de l'alcool enrichi en nitriles, qui est habituellement recyclé à la zone d'éthérification. Un tel procédé impose toutefois de coupler l'unité d'éthérification à une unité de craquage catalytique, ce qui peut s'avérer contraignant. Par ailleurs, l'alcool évacué vers l'unité de FCC est perdu, si bien que le bilan économique de cette opération est beaucoup moins intéressant que lorsque cet alcool est utilisé pour l'éthérification. Enfin, le reste d'alcool recyclé à l'unité d'éthérification présente toujours une teneur élevée en nitriles.

La présente invention vise à remédier à ces inconvénients, en proposant un procédé d'éthérification d'une charge d'hydrocarbures contenant des oléfines qui, contrairement aux procédés de l'art antérieur, ne nécessite pas de traitement préalable de la charge en amont de la zone d'éthérification, en vue d'en éliminer les composés azotés. A l'inverse, l'invention propose un traitement, en sortie de l'unité d'éthérification, de l'excès d'alcool résiduel, afin d'éliminer une fraction importante des composés azotés qui y sont présents, avant de recycler cet alcool au réacteur d'éthérification.

L'invention a également pour but de proposer un procédé de ce type, qui puisse être appliqué à toute charge d'hydrocarbures contenant des oléfines, qu'elle provienne ou non d'une unité F.C.C..

L'invention a enfin pour but un procédé de ce type, qui évite les phénomènes d'accroissement de la teneur en composés azotés dans le réacteur d'éthérification et augmente ainsi de manière très sensible la durée de vie du catalyseur, en diminuant fortement l'empoisonnement de celui-ci.

A cet effet, l'invention a pour objet un procédé d'éthérification d'oléfines comprenant :
- la mise en contact dans une zone réactionnelle, dans laquelle est présent un catalyseur de type acide et où règnent des conditions convenant à une réaction d'éthérification, d'au moins un alcool et d'une charge d'hydrocarbures riche en oléfines légères, mais contenant des composés azotés susceptibles de désactiver le catalyseur ;
- la séparation des effluents de la zone réactionnelle, de manière à obtenir au moins une première coupe riche en éther et une seconde coupe contenant la majeure partie de l'alcool résiduel et des composés azotés ;
- et le recyclage de la seconde coupe, à l'entrée de la zone réactionnelle ;
ce procédé étant caractérisé en ce que, préalablement à son recyclage dans la zone réactionnelle, la seconde coupe est soumise à un traitement comportant au moins une étape de mise en contact avec une masse absorbante acide, de manière à abaisser d'au moins 50 % sa concentration en lesdits composés azotés.

De préférence, le traitement de la seconde coupe, préalablement à son introduction dans la zone réactionnelle, est conduit dans des conditions telles que sa teneur en lesdits composés azotés est abaissée à une valeur inférieure ou égale à 10 p.p.m. et, de préférence, inférieure ou égale à 1 p.p.m.

La charge d'hydrocarbures riche en oléfines légères peut contenir avantageusement des oléfines comprenant de 2 à 10 atomes de carbone et, préférentiellement de 3 à 5 atomes de carbone.

Avant son recyclage à la zone réactionnelle, la seconde coupe est traitée de manière à en éliminer au moins 50 % des composés azotés, par mise en contact avec une masse adsorbante acide. Cette méthode de purification du recycle d'alcool est d'autant plus efficace qu'elle permet de n'extraire sélectivement que les composés nuisibles, c'est-à-dire les impuretés azotées, sans perte en alcool, celui-ci étant recyclé en totalité vers le réacteur d'éthérification.

Avantageusement, le traitement de cette seconde coupe comprend une première étape d'hydrogénation sélective des composés azotés qu'elle contient, notamment des nitriles, suivie d'une seconde étape d'adsorption, sur une masse adsorbante acide des composés azotés convertis par la phase d'hydrogénation.

L'étape d'hydrogénation sélective peut être conduite en particulier en présence d'hydrogène et d'un catalyseur hydrogénant. Divers catalyseurs, connus en soi, peuvent être employés à cet effet. On citera en particulier des catalyseurs comprenant au moins un métal de transition déposé sur un support d'oxyde minéral réfractaire. Le métal de transition est avantageusement choisi parmi les métaux du Groupe VIII de la Classification Périodique des Eléments (en particulier le nickel, le platine, le palladium, le ruthénium, le cobalt), le cuivre, l'argent, le zinc et leurs mélanges.

Cette étape d'hydrogénation des composés azotés, préalablement à leur extraction partielle de la seconde coupe, rend cette extraction plus facile. En effet, l'hydrogénation a pour effet de saturer les composés azotés insaturés présents, en particulier les nitriles, ce qui accroît leur caractère basique, et donc de favoriser leur rétention sur la masse adsorbante acide.

Les conditions opératoires de cette étape d'hydrogénation peuvent être les suivantes :
- vitesse spatiale horaire (en volume de charge liquide par unité de volume de catalyseur et par heure) : de 1 à 100 h⁻¹, de préférence de 10 à 50 h⁻¹ et, encore plus préférablement, de 10 à 20 h⁻¹ ;
- température (en °C) : de 0 à 200, de préférence de 0 à 100, encore plus préférablement de 0 à 30 ;
- pression (en bars) : de 1 à 40, de préférence de 1 à 25, encore plus préférablement de 5 à 20 ;
- rapport molaire hydrogène/composés azotés : de 2 à 250, de préférence de 5 à 100 et, encore plus préférablement de 10 à 50.

Que le procédé conforme à l'invention comporte ou non une étape d'hydrogénation sélective, l'étape d'élimination des composés azotés s'effectue avantageusement par extraction, par mise en contact de la seconde coupe avec une masse adsorbante acide à forte capacité d'échange. Comme masse adsorbante acide, on peut utiliser, en particulier, mais non limitativement :
- des silicoaluminates microporeux tels que les silicalites, les mordénites, les zéolites X et, de préférence, des zéolites Y,
- des résines macroréticulaires, greffées par des groupements acides (par exemple, acide sulfonique),
- de la silice imprégnée d'acide (par exemple, acide phosphorique ou acide sulfurique),
- du charbon actif, tel quel ou imprégné d'acide (par exemple, acide phosphorique ou acide sulfurique),
- de l'alumine activée,
- des argiles, telles quelles ou traitées par un acide,
- des tamis moléculaires,
- des aluminophosphates cristallins et microporeux,
- des silices-alumines mésoporeuses.

Outre leur efficacité pour la rétention des composés azotés, de telles masses adsorbantes présentent l'avantage d'être facilement régénérables, par simple chauffage à des températures de l'ordre de 400°C.

Le procédé conforme à l'invention diffère donc sur un point essentiel de la technique antérieure, puisque, contrairement à ce qu'enseigne celle-ci, il est tout-à-fait possible de laisser dans la charge d'oléfines à éthérifier tous les composés azotés qui y sont présents, ces composés n'étant extraits qu'ensuite, à raison d'au moins 50 %, de la seconde coupe riche en alcool, avant recyclage de celle-ci à l'entrée de la zone réactionnelle.

Ce mode de traitement du recycle d'alcool, et non de la charge à traiter, présente de nombreux avantages :
- il est plus facile à mettre en oeuvre, car les alcools sont beaucoup moins réactifs que les oléfines ; il est donc préférable de traiter spécifiquement le recycle d'alcool, sans risquer de dégrader les composés valorisables de la charge ; en outre, il est beaucoup plus difficile d'éliminer des impuretés azotées très diluées dans la charge d'oléfines que d'extraire 50% de ces mêmes impuretés, lorsque leur concentration est plus élevée (ce qui est le cas dans le recycle d'alcool) ;
- ce procédé est plus efficace, puisque, comme il est impossible d'éliminer totalement les composés azotés de la charge, dans les procédés de l'art antérieur, il en reste nécessairement dans le recycle d'alcool de sorte que, ces composés étant indéfiniment recyclés, leur teneur dans le recycle d'alcool ne peut manquer de s'accroître, avec pour conséquence une désactivation du catalyseur ;
- ce procédé est en outre moins coûteux, puisque l'on traite un recycle à faible débit et non la totalité du flux d'hydrocarbures pénétrant dans l'unité d'éthérification ; la capacité de traitement requise est donc réduite et les investissements et frais de fonctionnement sont nettement moindres.

Une forme de mise en oeuvre particulièrement avantageuse du procédé selon l'invention consiste à mettre en contact tout ou partie de la charge d'hydrocarbures riche en oléfines avec la seconde coupe, préalablement au traitement d'élimination des composés azotés de cette dernière. En effet, les composés azotés se sont avérés être plus solubles dans les alcools que dans les charges d'hydrocarbures oléfiniques, si bien qu'une telle mise en contact a pour effet de faire passer dans la seconde coupe une partie des composés azotés présents dans la charge. On récupère ainsi une charge d'oléfines appauvrie en impuretés azotées, et une seconde coupe enrichie en de telles impuretés, que l'on dirige alors vers le système d'élimination des composés azotés.

L'invention a également pour objet un dispositif comprenant :
- un réacteur dans lequel est présent un catalyseur de type acide et où règnent des conditions propres à une réaction d'éthérification d'oléfines en présence d'au moins un alcool ;
- au moins un conduit d'alimentation de ce réacteur en au moins un alcool et en au moins une charge d'hydrocarbures riche en oléfines légères et contenant des composés azotés ;
- un conduit d'évacuation des effluents du réacteur en direction d'au moins un moyen de séparation de ces effluents en une première coupe riche en éther et en une seconde coupe contenant la majeure partie de l'alcool résiduel et des composés azotés présents dans la charge d'hydrocarbures ;
- un circuit de recyclage de la seconde coupe depuis le moyen de séparation des effluents jusqu'à l'alimentation du réacteur d'éthérification ;
ce dispositif étant caractérisé en ce que le circuit de recyclage de la seconde coupe comprend, en amont de l'alimentation du réacteur d'éthérification, au moins une enceinte de traitement de cette seconde coupe contenant une masse absorbante acide à forte capacité d'échange, de manière à abaisser d'au moins 50 % la teneur en composés azotés de la seconde coupe.

Le procédé et le dispositif conformes à l'invention vont être décrits ci-après plus en détail dans leur application à la préparation de MTBE (méthyl tert-butyl-éther), mais ils ne sont naturellement pas limités à cette application, et ils peuvent aussi bien être utilisés pour la préparation d'ETBE (éthyl tert-butyl éther), de TAME (tert-amyl éther) ou de tout autre éther.

Dans la description qui va suivre, on se référera aux dessins annexés, sur lesquels :
La figure 1 est une vue schématique d'un appareillage utilisable pour la préparation de M.T.B.E., par le procédé d'éthérification conforme à l'invention ;
La figure 2 est une vue schématique d'une variante de l'appareillage de la figure 1.

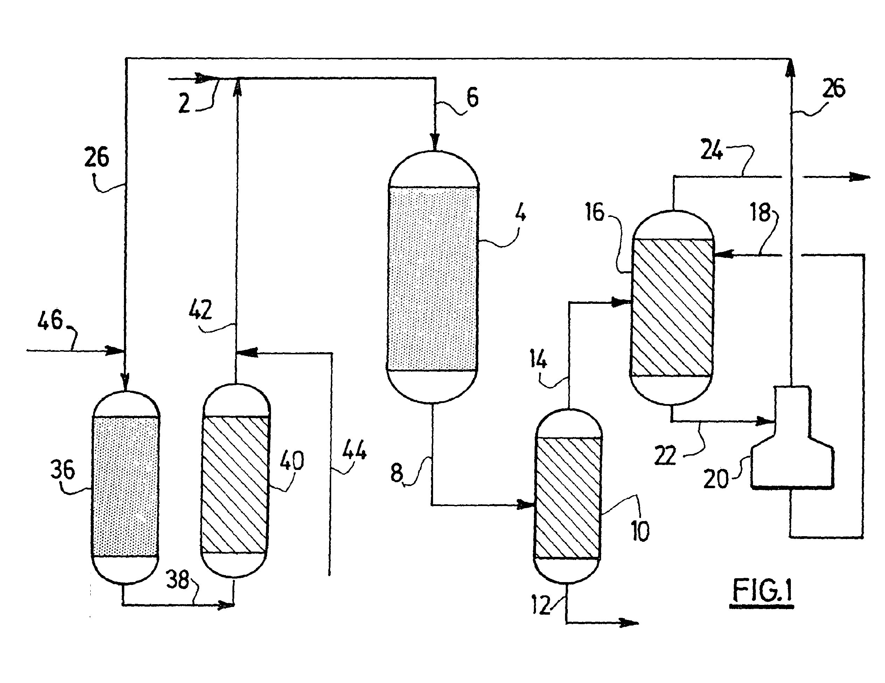

On se réfèrera d'abord à la figure 1, qui illustre la préparation de M.T.B.E. par réaction d'isobutène avec du méthanol en présence d'un catalyseur à sites acides, par exemple une résine constituée d'un copolymère de styrène et de divinyl benzène, greffée par des groupes acide sulfonique.

L'appareillage utilisé comprend un réacteur 4 d'éthérification, qui contient un lit du catalyseur mentionné ci-dessus, et qui est alimenté par la ligne 6 en un mélange d'une charge d'hydrocarbures, riche en isobutène, arrivant par la ligne 2, et de méthanol, arrivant par la ligne 42. La charge d'hydrocarbures comprend des impuretés azotées telles que des nitriles.

Les effluents de la réaction d'éthérification, en particulier le MTBE, sont évacués du réacteur 4 par la ligne 8 vers le séparateur 10, qui, de façon connue, comprend un débutaniseur. Le MTBE séparé par distillation dans l'enceinte 10 est évacué à la base de celle-ci par la ligne 12, pour être récupéré, tandis qu'à la partie supérieure, le reste des effluents est acheminé par la ligne 14 vers un second séparateur 16, dans lequel ces effluents subissent un lavage à l'eau.

L'eau, introduite dans le séparateur 16 par la ligne 18, y circule de haut en bas, de manière que les composés du reste de l'effluent du réacteur 4 qui sont solubles dans l'eau, en particulier le méthanol, soient séparés et entraînés avec l'eau, le mélange étant évacué par la ligne 22 vers un strippeur 20.

Les composés insolubles dans l'eau, comprenant essentiellement de l'isobutène et d'autres hydrocarbures présents dans l'effluent du réacteur 4, sont évacués par une ligne 24 à la partie supérieure du séparateur 16.

Dans le strippeur 20, le méthanol est séparé de l'eau, qui est recyclée par le conduit 18 au séparateur 16, tandis que le méthanol à recycler est évacué du strippeur 20 par la ligne 26.

Dans un procédé usuel d'éthérification, le méthanol, dans lequel se retrouvent les nitriles provenant de la charge d'oléfines traitée dans le réacteur 4 (tels quels ou sous forme d'autres composés azotés résultant de l'hydrolyse et/ou de l'alcoolyse de certains de ces nitriles dans le réacteur), est réintroduit directement dans la ligne 6 alimentant le réacteur 4.

Conformément à la présente invention toutefois, avant d'être réintroduit dans le réacteur, le recycle de méthanol est traité en vue d'en éliminer 50 % au moins des nitriles et autres impuretés azotées susceptibles de désactiver les sites acides du catalyseur présent dans le réacteur 4.

A cet effet, dans la forme de mise en oeuvre illustrée par la présente figure, le recycle de méthanol est transféré par la ligne 26 à un réacteur d'hydrogénation 36, contenant un lit de catalyseur d'hydrogénation, constitué, par exemple, par du nickel ou du palladium déposé sur un support minéral tel que de la silice ou de l'alumine, en présence duquel les composés azotés insaturés (en particulier les nitriles) présents dans le méthanol recyclé sont convertis en amines et/ou en imines. Cette conversion s'effectue en présence d'un gaz riche en hydrogène, introduit par la ligne 46 dans le flux de méthanol en amont du réacteur 36.

Le flux de méthanol ainsi traité est évacué à la base du réacteur 36 d'hydrogénation, par une ligne 38, vers une enceinte 40, qui contient une masse adsorbante acide apte à adsorber les composés azotés présents.

Dans le flux de méthanol purifié sortant par la ligne 42 de l'enceinte 40, est alors incorporé un appoint de méthanol frais, arrivant par la ligne 44, et le mélange est introduit dans la ligne 6 d'alimentation du réacteur d'éthérification 4.

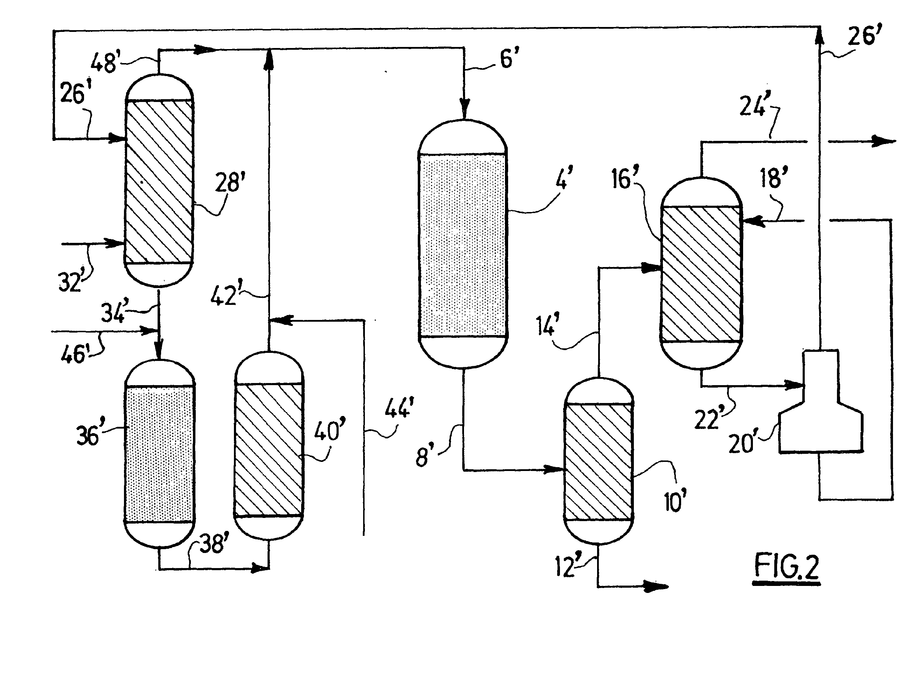

Dans la variante de mise en oeuvre du procédé conforme à l'invention illustrée par la figure 2, la charge d'isobutène, qui contient des impuretés azotées et notamment des nitriles, subit une phase préliminaire de lavage par le recycle de méthanol, avant le traitement de ce méthanol destiné à éliminer une fraction des composés azotés qu'il contient.

Sur cette figure 2, les constituants déjà décrits en référence à la figure 1 sont désignés par les mêmes chiffres de référence affectés de l'indice ' et ils ne seront donc pas décrits à nouveau.

On voit que le méthanol recyclé par la ligne 26' n'est plus introduit directement dans le réacteur 36' d'hydrogénation, mais alimente une enceinte 28', dans laquelle il circule de haut en bas, à contre-courant de la charge fraîche d'isobutène contenant des impuretés azotées, qui y est introduite par la ligne 32'.

Cette mise en contact, dans l'enceinte 28', du méthanol de recycle avec la charge fraîche d'isobutène, a pour effet d'extraire de cette dernière une partie des composés azotés qu'elle contient.

Dans le cas d'une unité de synthèse de MTBE telle que décrite ici, l'enceinte 28' peut être avantageusement constituée d'une colonne de distillation.

Toutefois, l'enceinte 28' peut être constituée de tout dispositif permettant d'effectuer une mise en contact intime, puis une séparation, du flux d'alcool recyclé (seconde coupe) et de la charge d'hydrocarbures riche en oléfines légères. En particulier, lorsque l'alcool est suffisamment peu soluble dans la charge d'oléfines légères, l'enceinte 28' peut être constituée d'une colonne de lavage à contre-courant.

Dans l'unité représentée ici, l'enceinte 28' est positionnée entre le strippeur 20' et le réacteur 36'. Toutefois, cette enceinte 28' peut parfaitement être positionnée à un autre endroit sur le circuit de traitement et de recyclage de l'alcool, pourvu qu'elle soit située en amont du système (36' ; 40') d'élimination des composés azotés. En particulier, l'enceinte 28' peut avantageusement être située entre le séparateur 16' et le strippeur 20'.

Le flux de recycle de méthanol, ainsi enrichi en composés azotés, sort au bas de l'enceinte 28' par la ligne 34', qui l'achemine vers l'unité d'hydrogénation 36' décrite ci-dessus. Quant à la charge fraîche d'isobutène appauvrie en composés azotés, elle est soutirée de l'enceinte 28' par la ligne 48', qui l'achemine vers la ligne 6' d'alimentation du réacteur d'éthérification 4'.

On notera qu'en raison de la teneur élevée en composés azotés du recycle de méthanol, il est plus facile d'hydrogéner ces composés dans le recycle que dans la coupe d'isobutène de départ, où leur teneur est beaucoup plus faible. En procédant à une telle hydrogénation dans la coupe d'isobutène, il y aurait d'ailleurs nécessairement hydrogénation d'une partie de l'isobutène, d'où une perte en cette matière première particulièrement coûteuse.

L'Exemple qui suit illustre certains des avantages apportés par la présente invention.

### EXEMPLE

### Test n°1 (selon l'invention) :

Dans une unité pilote de synthèse de MTBE conforme à la figure 1, on met en contact :
- une charge d'hydrocarbures oléfinique, comprenant 25 % en poids d'isobutène et un taux d'impuretés azotées de 2 p.p.m. en poids (essentiellement sous forme de nitriles), introduite avec un débit de 100 kg/h;
- du méthanol, avec un débit total de 15 kg/h, dont 13,57 kg/h de méthanol frais, et 1,43 kg/h de méthanol de recycle.

Le réacteur d'éthérification 4 renferme un catalyseur d'éthérification classique, constitué d'un copolymère de styrène et de vinyl-benzène greffé par des groupements acide sulfonique. Le réacteur fonctionne dans des conditions usuelles, à savoir :
- température : 55°C,
- pression : 12.10⁵Pa,
- vitesse spatiale horaire liquide : 4 h⁻¹,
- rapport méthanol/isobutène : 1,05.

Dans ces conditions, le taux de conversion de l'isobutène est de 95 %.

Selon l'invention, préalablement à son recyclage dans la zone réactionnelle 4, ladite seconde coupe contenant la majeure partie de l'alcool résiduel et des composés azotés est traitée de manière à abaisser substantiellement sa teneur en composés azotés.

Pour cela, elle est acheminée par la ligne 26 vers le réacteur d'hydrogénation 36, qui renferme un catalyseur commercial classique à base de nickel métallique (50 % en poids) déposé sur un support de silice et d'alumine. Ce catalyseur a été chargé dans le réacteur sous forme préréduite. Il présente les caractéristiques suivantes : une surface spécifique de 150 m²/g, un volume poreux de 0,5 cm³/g et une densité apparente de 0,8 g/cm³.

Préalablement à son utilisation, le catalyseur a été activé in situ sous flux d'hydrogène, par chauffage depuis la température ambiante jusqu'à 250°C (avec une vitesse d'élévation de température de 10°C par heure), puis maintien de la température à 250°C pendant 3,5 heures.

Les conditions opératoires régnant dans le réacteur d'hydrogénation 36 sont les suivantes :
- pression : 15.10⁵ Pa,
- température : 25°C,
- vitesse spatiale horaire (en volume de charge liquide par unité de volume de catalyseur par heure) : 12h⁻¹,
- rapport volumique débit d'hydrogène / débit de méthanol : 3,3 Nm³/m³.

Les effluents du réacteur d'hydrogénation 36 sont ensuite dirigés par la ligne 38 vers l'enceinte d'adsorption 40, qui renferme un tamis moléculaire comprenant une zéolite ultra-stable de type NH4-Y, ayant un rapport molaire silice/alumine de 5,38 et une surface spécifique de 625 m²/g. Avant utilisation, cette zéolite a été calcinée à une température de 350°C pendant 3 heures. Elle peut être régénérée facilement, par chauffage à une température d'environ 400°C.

Les conditions opératoires régnant dans l'enceinte 40 sont les suivantes :
- pression : 15.10⁵ Pa,
- température : 25°C,
- vitesse spatiale horaire (en volume de charge liquide par unité de volume du catalyseur par heure) : 1,2 h⁻¹.

Pendant toute la durée du test (300 heures), on a mesuré régulièrement, à la sortie de l'enceinte d'adsorption 40, la teneur en composés azotés du flux de méthanol recyclé au réacteur (avant ajout de l'appoint de méthanol frais), ainsi que la teneur totale en composés azotés dans le réacteur d'éthérification 4.

Les valeurs mesurées, stables pendant tout le test, sont les suivantes :
- teneur en composés azotés du flux de méthanol recyclé : néant (en-dessous du seuil de détection),
- teneur totale en composés azotés dans le réacteur d'éthérification : 1,74 p.p.m. en poids.

### Test n°2 (comparatif)

On reproduit le test n°1, mais dans une unité de synthèse de MTBE du type de celles communément employées dans l'art antérieur, sans purification du recycle de méthanol, qui est introduit tel quel dans le réacteur d'éthérification, après ajout de l'appoint en méthanol frais. En d'autres termes, on emploie une unité conforme à celle de la figure 1, mais qui ne comporte pas les enceintes 36 et 40.

De façon analogue, pendant toute la durée du test (300 heures), on a mesuré régulièrement la teneur en composés azotés du flux de méthanol recyclé au réacteur (avant ajout de l'appoint en méthanol frais), ainsi que la teneur totale en composés azotés dans le réacteur d'éthérification 4.

Les valeurs mesurées, stables pendant tout le test, sont les suivantes :
- teneur en composés azotés du flux de méthanol recyclé : 210 p.p.m. en poids,
- teneur totale en composés azotés dans le réacteur d'éthérification : 4,35 p.p.m. en poids.

Ces résultats montrent que, par rapport à une unité d'éthérification classique (test n°2), dans l'unité d'éthérification conforme à l'invention (test n°1), le taux d'impuretés azotées dans le réacteur d'éthérification 4 est 2,5 fois moins important. En supposant que la désactivation du catalyseur d'éthérification soit uniquement due à la présence des composés azotés, on peut escompter un allongement substantiel de la durée de vie du catalyseur, puisque celle-ci sera également multipliée par 2,5.

## Revendications

1. Procédé d'éthérification d'oléfines comprenant :
- la mise en contact, dans une zone réactionnelle dans laquelle est présent un catalyseur de type acide et où règnent des conditions convenant à une réaction d'éthérification, d'au moins un alcool et d'une charge d'hydrocarbures riche en oléfines légères, mais contenant des composés azotés susceptibles de désactiver le catalyseur ;
- la séparation des effluents de la zone réactionnelle de manière à obtenir au moins une première coupe riche en éther et une seconde coupe contenant la majeure partie de l'alcool résiduel et des composés azotés ;
- et le recyclage de la seconde coupe à l'entrée de la zone réactionnelle ;
ce procédé étant **caractérisé en ce que**, préalablement à son recyclage dans la zone réactionnelle, la seconde coupe est soumise à un traitement comportant au moins une étape de mise en contact avec une masse adsorbante acide, de manière à abaisser d'au moins 50 % sa concentration en lesdits composés azotés.

2. Procédé selon la revendication 1, **caractérisé en ce que** le traitement de la seconde coupe, préalablement à son introduction dans la zone réactionnelle, est conduite dans des conditions telles que sa teneur en composés azotés soit inférieure ou égale à 10 p.p.m. et, de préférence, inférieure ou égale à 1 p.p.m.

3. Procédé selon l'une des revendications 1 et 2, **caractérisé en ce que** la masse adsorbante acide est choisie dans le groupe constitué par :
- des silicoaluminates microporeux, tels que les silicalites, les mordénites, les zéolites X et, de préférence, les zéolites Y,
- des résines macroréticulaires, greffées par des groupements acides (par exemple, acide sulfonique),
- de la silice imprégnée d'acide (par exemple, acide phosphorique ou acide sulfurique),
- du charbon actif, tel quel ou imprégné d'acide (par exemple acide phosphorique ou acide sulfurique),
- de l'alumine activée,
- des argiles, telles quelles ou traitées par un acide,
- des tamis moléculaires,
- des aluminophosphates cristallins et microporeux,
- des silices-alumines mésoporeuses.

4. Procédé selon l'une des revendications 1 à 3, **caractérisé en ce que** préalablement à sa mise en contact avec une masse adsorbante acide, la seconde coupe est soumise à une phase d'hydrogénation sélective des composés azotés qu'elle contient.

5. Procédé selon la revendication 4, **caractérisé en ce que** la phase d'hydrogénation sélective est conduite de façon connue en présence d'hydrogène et d'un catalyseur hydrogénant, notamment un catalyseur contenant au moins un métal de transition déposé sur un support d'oxyde minéral réfractaire.

6. Procédé selon la revendication 5, **caractérisé en ce que** le métal de transition est choisi parmi les métaux du Groupe VIII de la Classification Périodique des Eléments (en particulier le nickel, le platine, le palladium, le ruthénium, le cobalt), le cuivre, l'argent, le zinc et leurs mélanges.

7. Procédé selon l'une des revendications 4 à 6, **caractérisé en ce que** la phase d'hydrogénation sélective est mise en oeuvre dans les conditions suivantes :
- vitesse spatiale horaire (en volume de charge par unité de volume de catalyseur et par heure) : de 1 à 100h⁻¹, de préférence 10 à 50h⁻¹ et, encore plus préférablement, de 10 à 20 h⁻¹;
- température (en °C) : de 0 à 200, de préférence de 0 à 100, encore plus préférablement de 0 à 30;
- pression (en bars) : de 1 à 40, de préférence de 1 à 25, encore plus préférablement de 5 à 20 ;
- rapport molaire hydrogène/composés azotés : de 2 à 250, de préférence de 5 à 100 et, encore plus préférablement, de 10 à 50.

8. Procédé selon l'une des revendications 1 à 7, **caractérisé en ce que**, avant son introduction dans la zone réactionnelle d'éthérification, tout ou partie de la charge d'hydrocarbures riche en oléfines légères est mise en contact avec la seconde coupe, préalablement au traitement de cette seconde coupe destiné à abaisser d'au moins 50 % sa concentration en composés azotés.

9. Dispositif d'éthérification d'oléfines, ce dispositif comprenant :
- un réacteur (4) dans lequel est présent un catalyseur de type acide et où règnent des conditions propres à une réaction d'éthérification d'oléfines en présence d'au moins un alcool ;
- au moins un conduit (6) d'alimentation de ce réacteur en au moins un alcool et en au moins une charge d'hydrocarbures riche en oléfines légères et contenant des composés azotés ;
- un conduit (8) d'évacuation des effluents du réacteur en direction d'au moins un moyen (10; 16 ; 20) de séparation de ces effluents en une première coupe riche en éther et en une seconde coupe contenant la majeure partie de l'alcool résiduel et des composés azotés présents dans la charge d'hydrocarbures ;
- un circuit (26) de recyclage de la seconde coupe (26) depuis le moyen (10 ; 16; 20) de séparation des effluents jusqu'à l'alimentation du réacteur (4) d'éthérification ;
ce dispositif étant **caractérisé en ce que** le circuit de recyclage de la seconde coupe comprend, en amont de l'alimentation du réacteur (4), au moins une enceinte (40) de traitement de cette seconde coupe, contenant une masse adsorbante acide à forte capacité d'échange, de manière à abaisser d'au moins 50 % la teneur en composés azotés de la seconde coupe.

10. Dispositif selon la revendication 9, **caractérisé en ce que**, en amont de l'enceinte (40) de traitement de la seconde coupe, le circuit de recyclage de celle-ci comprend un réacteur (36) d'hydrogénation, ce réacteur (36) étant équipé d'une alimentation (46) en hydrogène et contenant un catalyseur d'hydrogénation, ce réacteur fonctionnant dans des conditions telles que les composés azotés de la seconde coupe y soient soumis au moins partiellement à une hydrogénation sélective.

11. Dispositif selon la revendication 9 **caractérisé en ce que** le circuit de recyclage de la seconde coupe à l'alimentation du réacteur (4') d'éthérification comprend, en amont de l'enceinte (40') d'élimination des composés azotés et, éventuellement, du réacteur (36') d'hydrogénation sélective des composés hydrogénés contenus dans la seconde coupe, une enceinte (28') alimentée en cette seconde coupe et en tout ou partie de la charge d'hydrocarbures à traiter, et dans laquelle sont effectuées une mise en contact intime, puis une séparation, de ladite seconde coupe et de tout ou partie de la charge d'hydrocarbures.

12. Utilisation du procédé selon l'une des revendications 1 à 8, ou du dispositif selon l'une des revendications 9 à 11, pour la préparation de méthyl tert-butyl éther (MTBE), d'éthyl tert-butyl éther (ETBE), de tert-amyl éther (TAME) ou de tout autre éther.

## Claims

1. A process for the etherification of olefins comprising:
- bringing into contact, in a reaction zone in which is present a catalyst of the acid type and in which prevail conditions suitable for an etherification reaction, at least one alcohol and a hydrocarbon charge rich in light olefins but containing nitrogen-containing compounds which are liable to deactivate the catalyst;
- separating the effluents from the reaction zone so as to obtain at least one first fraction rich in ether and a ' second fraction containing the major portion of the residual alcohol and nitrogen-containing compounds;
- and recycling the second fraction to the inlet of the reaction zone;
said process being **characterised in that**, prior to it being recycled into the reaction zone, the second fraction undergoes a treatment comprising at least one step of being brought into contact with an adsorbent acid mass so as to lower by at least 50 % its concentration of said nitrogen-containing compounds.

2. A process according to claim 1, **characterised in that** the treatment of the second fraction, prior to it being introduced into the reaction zone, is carried out under conditions such that its content of nitrogen-containing compounds is lower or equal to 10 p.p.m. and, preferably, lower or equal to 1 p.p.m.

3. A process according to either one of claims 1 and 2, **characterised in that** the adsorbent acid mass is chosen from the group formed by:
- microporous alumino-silicates, such as the silicalites, the mordenites, the X zeolites and, preferably, the Y zeolites,
- macroreticular resins grafted with acid groups (for example, sulphonic acid),
- silica impregnated with acid (for example, phosphoric acid or sulphuric acid),
- activated carbon, unmodified or impregnated with acid (for example, phosphoric acid or sulphuric acid),
- activated alumina;
- clays, unmodified se or treated with an acid,
- molecular sieves,
- crystalline and microporous aluminophosphates,
- mesoporous alumino-silicas.

4. A process according to any one of claims 1 to 3, **characterised in that**, prior to it being brought into contact with an adsorbent acid mass, the second fraction is subjected to a hydrogenation phase selective for the nitrogen-containing compounds which it contains.

5. A process according to claim 4, **characterised in that** the selective hydrogenation phase is carried out in a known manner in the presence of hydrogen and a hydrogenating catalyst, in particular a catalyst containing at least one transition metal deposited on a refractory mineral oxide support.

6. A process according to claim 5, **characterised in that** the transition metal is chosen from the metals of Group VIII of the periodic table of the elements (in particular nickel, platinum, palladium, ruthenium, cobalt), copper, silver, zinc and mixtures thereof.

7. A process according to any one of claims 4 to 6, **characterised in that** the selective hydrogenation phase is implemented under the following conditions:
- hourly spatial velocity (volume of charge per unit of volume of catalyst and per hour): from 1 to 100h⁻¹, preferably 10 to 50h⁻¹, and even more preferably, from 10 to 20h⁻¹;
- temperature (in °C): from 0 to 200, preferably from 0 to 100, even more preferably from 0 to 30;
- pressure (in bars): from 1 to 40, preferably from 1 to 25, even more preferably from 5 to 20;
- hydrogen/nitrogen-containing compounds molar ratio: from 2 to 250, preferably from 5 to 100 and, even more preferably, from 10 to 50.

8. A process according to any one of claims 1 to 7, **characterised in that**, prior to it being introduced into the etherification reaction zone, all or part of the hydrocarbon charge rich in light olefins is brought into contact with the second fraction, prior to the treatment of this second fraction intended to lower by at least 50 % its concentration of nitrogen-containing compounds.

9. An apparatus for the etherification of olefins, this apparatus comprising:
- a reactor (4) in which is present a catalyst of the acid type and in which prevail conditions suitable for an etherification reaction of olefins in the presence of at least one alcohol;
- at least one duct (6) for supplying this reactor with at least one alcohol and at least one hydrocarbon charge rich in light olefins and containing nitrogen-containing compounds;
- a duct (8) for discharging the effluents from the reactor zone towards at least one means (10;16;20) for separating these effluents into at least one first fraction rich in ether and into a second fraction containing the major portion of the residual alcohol and nitrogen-containing compounds present in the hydrocarbon charge;
- a circuit (26) for recycling the second fraction (26) from the means (10;16;20) for separating the effluents up to the feed of the etherification reactor (4);
said apparatus being **characterised in** the recycling circuit of the second fraction comprises, upstream of the feed of the reactor (4), at least one enclosure (40) for treating said second fraction, containing an adsorbent acid mass with a strong exchange capacity so as to lower by at least 50 % the content of nitrogen-containing compounds of the second fraction.

10. An apparatus according to claim 9, **characterised in that**, upstream of the treatment enclosure (40) for the second fraction, the recycling circuit of the latter comprises a hydrogenation reactor (36), said reactor (36) being provided with a hydrogen supply (46) and containing a hydrogenation catalyst, said reactor operating under conditions such that the nitrogen-containing compounds of the second fraction are subjected therein at least partly to a selective hydrogenation.

11. An apparatus according to claim 9, **characterised in that** the circuit for recycling the second fraction to the feed of the etherification reactor (4') comprises, upstream of the enclosure (40') for eliminating nitrogen-containing compounds and, optionally, of the reactor (36') for the selective hydrogenation of the nitrogen-containing compounds contained in the second fraction, an enclosure (28') supplied with said second fraction and all or part of the hydrocarbon charge to be treated, and in which are carried out intimate contact, then separation, of said second fraction and all or part of the hydrocarbon charge.

12. Use of the process according to any one of claims 1 to 8, or of the apparatus according to any one of claims 9 to 11, for the preparation of methyl tert-butyl ether (MTBE), ethyl tert-butyl ether (ETBE), tert-amyl methyl ether (TAME) or any other ether.

## Patentansprüche

1. Verfahren zur Etherifizierung von Olefinen, umfassend:
- in Kontakt bringen von mindestens einem Alkohol mit einer Kohlenwasserstoffbeladung, die reich an leichten Olefinen ist, aber Stickstoffverbindungen enthält, die imstande sind, den Katalysator zu desaktivieren, in einer Reaktionszone, in welcher ein Katalysator sauren Typs vorliegt und wo Bedingungen herrschen, die zu einer Etherifizierungsreaktion passen.
- die Trennung des Ausstroms der Reaktionszone derart, dass mindestens ein erster Verschnitt, der reich an Ether ist, und ein zweiter Verschnitt, der den Hauptteil von restlichem Alkohol und Stickstoffverbindungen enthält, erhalten werden;
- und das Rezyklisieren des zweiten Verschnitts zum Eintritt in die Reaktionszone; wobei das Verfahren **dadurch gekennzeichnet ist, dass** der zweite Verschnitt vor seinem Rezyklisieren in der Reaktionszone einer Behandlung unterzogen wird, die mindestens eine Stufe des in Kontakt Bringens mit einer Masse sauren Adsorptionsmittels umfasst, derart, dass seine Konzentration an besagten Stickstoffverbindungen um mindestens 50% vermindert wird.

2. Verfahren gemäß Anspruch 1, **dadurch gekennzeichnet, dass** die Behandlung des zweiten Verschnitts vor seiner Einführung in die Reaktionszone unter solchen Bedingungen gesteuert wird, dass sein Gehalt an Stickstoffverbindungen kleiner oder gleich 10 ppm und vorzugsweise kleiner oder gleich 1 ppm ist.

3. Verfahren gemäß einem der Ansprüchen 1 und 2, **dadurch gekennzeichnet, dass** die Masse sauren Adsorptionsmittels ausgewählt wird aus der Gruppe bestehend aus:
- mikroporösen Aluminosilikaten, wie Silikalite, Mordenite, X-Zeolithe und vorzugsweise Y-Zeolithe,
- makronetzartige Harze, aufgepfropft mit Säuregruppen (zum Beispiel Sulfonsäure),
- Siliciumdioxid, getränkt mit Säure (zum Beispiel Phosphorsäure oder Schwefelsäure),
- Aktivkohle, wie sie ist oder mit Säure getränkt (zum Beispiel Phosphorsäure oder Schwefelsäure),
- aktiviertes Aluminiumoxid,
- Ton, wie er ist oder mit einer Säure behandelt,
- Molekularsiebe,
- kristalline und mikroporöse Aluminiumphosphate mesoporöse Siliciumdioxid-Aluminiumoxide.

4. Verfahren gemäß einem der Ansprüche 1 bis 3, **dadurch gekennzeichnet, dass** der zweite Verschnitt, vor seinem in Kontakt Bringen mit einer Masse sauren Adsorptionsmittels, einer Hydrierungsphase unterworfen wird, die selektiv bezüglich der Stickstoffverbindungen ist, die dieser enthält.

5. Verfahren gemäß Anspruch 4, **dadurch gekennzeichnet, dass** die selektive Hydrierungsphase auf bekannte Art und Weise unter Anwesenheit von Wasserstoff und einem Hydrierkatalysator durchgeführt wird, insbesondere einem Katalysator, der mindestens ein Übergangsmetall enthält, das auf einem Trägermaterial aus hitzebeständigem mineralischem Oxid abgeschieden ist.

6. Verfahren gemäß Anspruch 5, **dadurch gekennzeichnet, dass** das Übergangsmetall aus der Gruppe VIII des Periodensystems der Elemente (insbesondere Nickel, Platin, Palladium, Ruthenium, Kobalt), Kupfer, Silber, Zink und deren Mischungen, ausgewählt wird.

7. Verfahren gemäß einem der Ansprüche 4 bis 6, **dadurch gekennzeichnet, dass** die selektive Hydrierungsphase unter den folgenden Bedingungen durchgeführt wird:
- Umsatzgeschwindigkeit (in Beladungsvolumen pro Volumeneinheit Katalysator pro Stunde): von 1 bis 100 h⁻¹, vorzugsweise 10 bis 50 h⁻¹ und, noch weiter bevorzugt 10 bis 20 h⁻¹ ;
- Temperatur (in °C): von 0 bis 200, vorzugsweise 0 bis 100, noch weiter bevorzugt 0 bis 30;
- Druck (in bar): von 1 bis 40, vorzugsweise 1 bis 25, noch weiter bevorzugt 5 bis 20;
- molares Verhälnis Wasserstoff/Stickstoffverbindungen: von 2 bis 250, vorzugsweise 5 bis 100 und, noch weiter bevorzugt 10 bis 50.

8. Verfahren gemäß einem der Ansprüche 1 bis 7, **dadurch gekennzeichnet, dass** alles oder ein Teil der Kohlenwasserstoffbeladung, die reich an leichten Olefinen ist, vor ihrer Einleitung in die Etherifzierungs-Reaktionszone in Kontakt gebracht wird mit dem zweiten Verschnitt vor der Behandlung von diesem zweiten Verschnitt, die zur Verringerung von dessen Konzentration an Stickstoffverbindungen um mindestens 50 % bestimmt ist.

9. Vorrichtung zur Etherifizierung von Olefinen, wobei diese Vorrichtung umfasst:
- einen Reaktor (4), in welchem ein Katalysator sauren Typs vorliegt und wo geeignete Bedingungen für eine Etherifizierung von Olefinen in Gegenwart von mindestens einem Alkohol herrschen;
- mindestens eine Leitung (6) zur Einspeisung von mindestens einem Alkohol und mindestens einer Kohlenwasserstoffbeladung, die reich an leichten Olefinen ist und Stickstoffverbindungen enthält, in diesen Reaktor;
- eine Leitung (8) zum Abführen des Reaktorausstroms in Richtung mindestens einer Einrichtung (10; 16; 20) zur Trennung von diesem Ausstrom in einen ersten Verschnitt, der reich an Ether ist, und einen zweiten Verschnitt, der den Hauptteil des restlichen Alkohols und Stickstoffverbindungen enthält, die in der Kohlenwasserstoffbeladung vorliegen;
- ein Rezyklisierungskreislauf (26) des zweiten Verschnitts (26) von der Einrichtung (10; 16; 20) zur Trennung von diesem Ausstrom bis zur Einspeisung in den Etherifizierungs-Reaktor (4);
wobei diese Vorrichtung **dadurch gekennzeichnet ist, dass** der Rezyklisierungskreislauf des zweiten Verschnitts stromaufwärts der Einspeisung in den Reaktor (4) mindestens einen Raum (40) zur Behandlung dieses zweiten Verschnitts umfasst, der eine Masse sauren Adsorptionsmittels mit hoher Austauschkapazität enthält, um den Gehalt an Stickstoffverbindungen in dem zweiten Verschnitt um mindestens 50% zu verringern.

10. Vorrichtung gemäß Anspruch 9, **dadurch gekennzeichnet, dass** - stromaufwärts des Raums (40) zur Behandlung des zweiten Verschnitts - der Kreislauf zu dessen Rezyklisierung einen Hydrierreakor (36) umfasst, wobei dieser Reaktor (36) ausgestattet ist mit einer Einspeisung (46) für Wasserstoff und einen Hydrierkatalysator enthält, wobei dieser Reaktor unter Bedingungen arbeitet, unter denen die Stickstoffverbindungen des zweiten Verschnitts dort mindestens teilweise einer selektiven Hydrierung unterworfen werden.

11. Vorrichtung gemäß Anspruch 9, **dadurch gekennzeichnet, dass** der Kreislauf zur Rezyklisierung des zweiten Verschnitts zur Einspeisung in den Etherifizierungsreaktor (4') - stromaufwärts des Raums (40') zur Eliminierung von Stickstoffverbindungen und gegebenenfalls des Reaktors (36') zur selektiven Hydrierung der in dem zweiten Verschnitt enthaltenen Stickstoffverbindungen - einen Raum (28') zum Beschicken dieses zweiten Verschnitts und des Gesamten oder einem Teil der zu behandelnden Kohlenwasserstoffbeladung umfasst, und in welchem ein inniges in Kontakt Bringen, dann eine Abtrennung des besagten zweiten Verschnitts und dem Gesamten oder einem Teil der Kohlenwasserstoffbeladung bewirkt werden.

12. Verwendung des Verfahrens gemäß einem der Ansprüche 1 bis 8, oder der Vorrichtung gemäß einem der Ansprüche 9 bis 11, zur Herstellung von Methyl-tert-butylether (MTBE), Ethyltert-butylether (ETBE), *tert*-Amylmethylether (TAME) oder von anderen Ethern.
